# EUROPEAN PATENT SPECIFICATION

(11) **EP 2 327 448 B1**
(45) Date of publication and mention of the grant of the patent: **19.10.2016**
(21) Application number: 09812989.3
(22) Date of filing: 24.08.2009
(51) Int. Cl.: A61B 5/042, A61N 1/05, A61N 1/39

(54) **DEFIBRILLATION CATHETER**
DEFIBRILLATIONSKATHETER
CATHÉTER DE DÉFIBRILLATION

(30) Priority: 11.09.2008 JP 2008233742
(43) Date of publication of application: 01.06.2011
(73) Proprietor: Japan Lifeline Co., Ltd., Tokyo 140-0002 (JP)
(72) Inventor: MORI, Kenji, Tokyo 140-0002 (JP); SAKANO, Yasuo, Tokyo 140-0002 (JP)
(74) Representative: Grünecker Patent- und Rechtsanwälte PartG mbB
(86) International application number: PCT/JP2009/064693
(87) International publication number: WO 2010/029842

(56) References cited:
- WO-A2-2005/002665
- JP-A- 2006 255 401
- JP-T- 2002 531 164
- JP-T- 2007 521 076
- JP-T- 2008 523 950
- US-A- 5 697 965
- US-A1- 2005 113 897
- US-A1- 2006 184 106
- US-B1- 6 178 355
- US-B1- 6 201 994

## Description

### TITLE OF INVENTION DEFIBRILLATION CATHETER

### TECHNICAL FIELD

The present invention relates to an intracardiac defibrillation catheter inserted into a cardiac cavity to remove atrial fibrillation.

### BACKGROUND ART

An automated external defibrillator (AED) is known as a defibrillator for removing atrial fibrillation (see, for example, Patent Literature 1).

In a defibrillation treatment by the AED, an electrode pad is attached to the body surface of a patient to apply a direct current voltage thereto, thereby giving electric energy within the body of the patient. Here, the electric energy flowing within the body of the patient from the electrode pad is generally controlled to 150 to 200 J, and a part (generally, about several % to 20%) thereof flows into a heart to be used for defibrillation treatment.

The state of the art disclosed in US 2005/0113897 A1 refers to an implantable medical lead including a stimulating electrode and a physiological sensor arranged along a body of the lead. According to US 2005/0113897 A1, the electrical lead may comprise a first defibrillation electrode and a second defibrillation electrode. Further to these defibrillation electrodes, the implantable electrical lead may also be equipped with an anode ring electrode and a cathode tip electrode at the distal tip of the lead, whereas the anode ring electrode and the cathode tip electrode are arranged for pacing a heart or sensing the electrical activity of a heart.

Document US 6,201,994 B1 discloses an implantable cardiac pacing lead for pacing the atria in the method of its use. The lead is provided with a mechanism for maintaining an atrial pacing electrode adjacent stimulable tissue in a patient's superior vena cava and an additional electrode locatable in the right ventricle or elsewhere when the atrial electrode is so located. Thereby, the implantable lead shown in US 6,201,995 may comprise a pacing electrode at a distal end and two pacing electrodes in a proximal region of the lead. One or more of the pacing electrodes may be substituted by a defibrillation electrode.

The prior art in US 2006/0184106 A1 discloses an improved steerable catheter with in-plane deflection comprising a catheter body having proximal and distal ends and a lumen extending there through and a tip section at the distal end of the catheter body. Said steerable catheter comprises three ring electrodes and one tip electrode. US 5,697,965 discloses an intracardiac defibrillation catheter according to the first part of claim 1.

### PRIOR ART LITERATURE

### PATENT LITERATURE

Patent Literature 1: Japanese Patent Application Laid-Open No. 2001-112874

### SUMMARY OF THE INVENTION

### PROBLEM TO BE SOLVED BY THE INVENTION

Atrial fibrillation is liable to occur during cardiac catheterization, and it is necessary to conduct electrical defibrillation even in this case.

According to the AED that electric energy is supplied from the outside of the body, however, it is difficult to supply effective electric energy (for example, 10 to 30 J) to a heart that has suffered the atrial fibrillation.

In other words, when a proportion of electric energy flowing into the heart of the electric energy supplied from the outside of the body is small (for example, about several %), it is impossible to conduct a sufficient defibrillation treatment.

On the other hand, when the electric energy supplied from the outside of the body flows into the heart in a high proportion, it is considered that the tissue of the heart may possibly be damaged.

In addition, in the defibrillation treatment by the AED, burn is easy to occur on the body surface to which the electrode pad has been attached. When the proportion of the electric energy flowing into the heart is small as described above, the degree of burn becomes heavy by supplying the electric energy repeatedly to be a considerable burden on the patient subjected to the catheterization.

The present invention has been made on the basis of the foregoing circumstances and has as an obj ect the provision of an intracardiac defibrillation catheter capable of surely supplying electric energy necessary and sufficient for defibrillation to a heart that has suffered atrial fibrillation during cardiac catheterization.

Another object of the present invention is to provide an intracardiac defibrillation catheter by which a defibrillation treatment can be conducted without causing burn on the body surface of a patient.

### MEANS FOR SOLVING PROBLEM

(1) The intracardiac defibrillation catheter according to the present invention is a catheter as defined in claim 1.

The intracardiac defibrillation catheter of such a structure is inserted into a cardiac cavity in such a manner that the first DC electrode group is located in a coronary vein, and the second DC electrode group is located in a right atrium to respectively apply voltages different in polarity from each other to the first DC electrode group and the second DC electrode group (apply a direct current voltage between the first DC electrode group and the second DC electrode group) through the first lead wire group and the second lead wire group, thereby directly applying electric energy to a heart that has suffered fibrillation to conduct a defibrillation treatment.

As described above, the electric energy is directly given to the heart that has suffered the fibrillation by the first DC electrode group and the second DC electrode group of the defibrillation catheter arranged within the cardiac cavity, whereby electric stimulus (electric shock) necessary and sufficient for defibrillation treatment can be surely given only to the heart.

In addition, no burn is caused on the body surface of the patient because the electric energy can be directly applied to the heart.

The first lead wire group composed of lead wires connected to the respective electrodes making up the first DC electrode group, and the second lead wire group composed of lead wires connected to the respective electrodes making up the second DC electrode group respectively extend into the different lumens of the tube member, whereby both lead wire groups are completely insulated and isolated from each other within the tube member. Thus, occurrence of short circuit between the first lead wire group (first DC electrode group) and the second lead wire group (second DC electrode group) within the tube member can be surely prevented when the voltage necessary for the intracardiac defibrillation is applied.
(2) In the intracardiac defibrillation catheter it may be preferable that the catheter comprises
   a potential-measuring electrode group composed of a plurality of electrodes installed on the tube member apart from the first DC electrode group or the second DC electrode group, and
   a lead wire group for potential measurement, which is composed of lead wires connected to the respective electrodes making up the potential-measuring electrode group, wherein
   the lead wire group for potential measurement extends into any other lumen of the tube member than both lumens into which the first lead wire group and the second lead wire group extend.

According to the intracardiac defibrillation catheter of such a structure, a cardiac potential can be measured by the potential-measuring electrode group to conduct the defibrillation treatment while monitoring the cardiac potential.

The lead wire group for potential measurement extends into any other lumen of the tube member than both lumens into which the first lead wire group and the second lead wire group extend, whereby the lead wire group for potential measurement is completely insulated and isolated from both the first lead wire group and the second lead wire group. Thus, occurrence of short circuit between the lead wire group for potential measurement (potential-measuring electrode group) and the first lead wire group or second lead wire group (first DC electrode group or second DC electrode group) within the tube member can be surely prevented when the voltage necessary for the intracardiac defibrillation is applied.
(3) The intracardiac defibrillation catheter according to the present invention further comprises
   a distal-side potential-measuring electrode group composed of a plurality of electrodes and installed on the tube member towards distal direction from the first DC electrode group,
   a proximal-side potential-measuring electrode group composed of a plurality of ring-like electrodes and installed on the tube member towards proximal direction from the second DC electrode group,
   a third lead wire group composed of lead wires connected to the respective electrodes making up the distal-side potential-measuring electrode group, and
   a fourth lead wire group composed of lead wires connected to the respective electrodes making up the proximal-side potential-measuring electrode group, wherein
   the third lead wire group and the fourth lead wire group extend into any other lumen of the tube member than both lumens into which the first lead wire group and the second lead wire group extend.

According to the intracardiac defibrillation catheter of such a structure, a cardiac potential can be measured by the distal-side potential-measuring electrode group and the proximal-side potential-measuring electrode group to conduct the defibrillation treatment while monitoring the cardiac potential.

The third lead wire group and the fourth lead wire group extend into any other lumen of the tube member than both lumens into which the first lead wire group and the second lead wire group extend, whereby the third lead wire group and the fourth lead wire group are completely insulated and isolated from both the first lead wire group and the second lead wire group. Thus, occurrence of short circuit between the third lead wire group or fourth lead wire group (distal-side potential-measuring electrode group or proximal-side potential-measuring electrode group) and the first lead wire group or second lead wire group (first DC electrode group or second DC electrode group) within the tube member can be surely prevented when the voltage necessary for the intracardiac defibrillation is applied.
(4) In the intracardiac defibrillation catheter of (3), it may be preferable that
   4 lumens are formed in the tube member,
   the first lead wire group extends into a first lumen,
   the second lead wire group extends into a second lumen,
   the third lead wire group and fourth lead wire group extend into a third lumen, and
   a pull wire for distal end deflection operation extends into a fourth lumen.

According to the intracardiac defibrillation catheter of such a structure, occurrence of short circuit among the first lead wire group (first DC electrode group), the second lead wire group (second DC electrode group) and the third lead wire group or fourth lead wire group (distal-side potential-measuring electrode group or proximal-side potential-measuring electrode group) can be surely prevented. Since the pull wire for distal end deflection operation extends into any other lumen than the lumens into which the respective lead wire groups extend, the lead wires making up the lead wire groups are not damaged (for example, abraded) by the pull wire moving in an axial direction upon a distal end deflection operation.
(5) In the intracardiac defibrillation catheter according to the present invention, it may be preferable that the first lead wire group and the second lead wire group extend into inner holes of insulated tubes different from each other in the interior of the handle.

According to the intracardiac defibrillation catheter of such a structure, the first lead wire group and the second lead wire group are completely insulated and isolated from each other even in the interior of the handle. Thus, occurrence of short circuit between the first lead wire group and the second lead wire group in the interior of the handle can be surely prevented when the voltage necessary for the intracardiac defibrillation is applied.
(6) In the intracardiac defibrillation catheter of (4), it may be preferable that in the interior of the handle, the first lead wire group extends into a first insulated tube connected to the first lumen,
   the second lead wire group extends into a second insulated tube connected to the second lumen, and
   the third lead wire group and the fourth lead wire group extend into a third insulated tube connected to the third lumen.

According to the intracardiac defibrillation catheter of such a structure, the first lead wire group, the second lead wire group, and the third lead wire group and the fourth lead wire group are completely insulated and isolated from one another even in the interior of the handle. Thus, occurrence of short circuit among the first lead wire group, the second lead wire group and the third lead wire group or the fourth lead wire group in the interior of the handle can be surely prevented when the voltage necessary for the intracardiac defibrillation is applied.
(7) The intracardiac defibrillation catheter according to the present invention may preferably be inserted into a cardiac cavity to remove atrial fibrillation caused during cardiac catheterization.

### EFFECTS OF INVENTION

According to the intracardiac defibrillation catheter of the present invention, electric energy necessary and sufficient for defibrillation can be surely supplied to a heart that has suffered atrial fibrillation during cardiac catheterization. In addition, no burn is caused on the body surface of a patient, and invasiveness is also little.

Further, occurrence of short circuit between the first lead wire group (first DC electrode group) and the second lead wire group (second DC electrode group) can be surely prevented when the voltage necessary for the intracardiac defibrillation is applied.

### BRIEF DESCRIPTION OF THE DRAWINGS

[FIG. 1] is an explanatory plan view illustrating an embodiment of an intracardiac defibrillation catheter according to the present invention.
[FIG. 2] is an explanatory plan view (drawing for explaining dimensions and hardnesses) illustrating the embodiment of the intracardiac defibrillation catheter according to the present invention.
[FIG. 3] is a cross-sectional view illustrating a section A-A in FIG. 1.
[FIG. 4] is cross-sectional views illustrating sections B-B, C-C and D-D in FIG. 1.
[FIG. 5] is a longitudinal sectional view (illustrating a section E-E in FIG. 3) in a distal region of a multi-lumen tube.

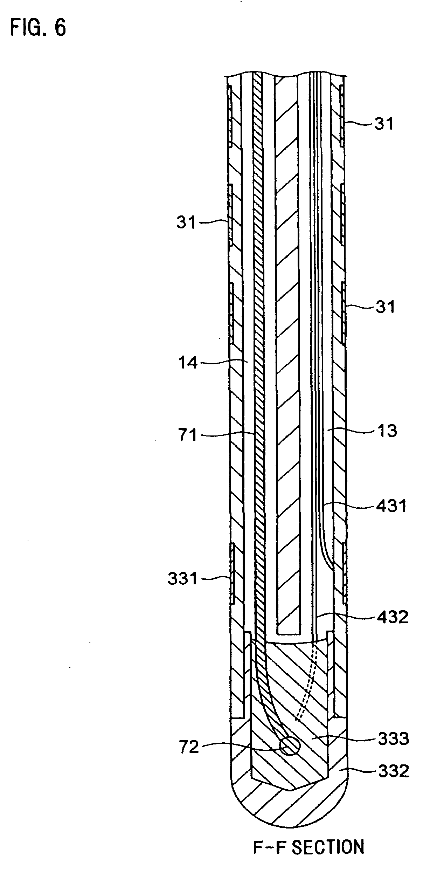

[FIG. 6] is a longitudinal sectional view (illustrating a section F-F in FIG. 3) in the distal region of the multi-lumen tube.
[FIG. 7] is a longitudinal sectional view (illustrating a section G-G in FIG. 3) in the distal region of the multi-lumen tube.
[FIG. 8] is a longitudinal sectional view (illustrating a section I-I in FIG. 3) in an intermediate region of the multi-lumen tube.
[FIG. 9] is an explanatory view typically illustrating the interior of a handle making up the intracardiac defibrillation catheter according to the present invention.
[FIG. 10] is an explanatory view illustrating, on an enlarged scale, a proximal end portion of the multi-lumen tube connected to the handle.
[FIG. 11] is a potential waveform diagram measured when predetermined electric energy has been applied by the intracardiac defibrillation catheter according to the present invention.
[FIG. 12] is cross-sectional views illustrating modified examples of the intracardiac defibrillation catheter according to the present invention.

### MODE FOR CARRYING OUT THE INVENTION

An embodiment of the intracardiac defibrillation catheter according to the present invention will hereinafter be described.

The intracardiac defibrillation catheter 100 according to this embodiment is equipped with a multi-lumen tube 10, a handle 20, a first DC electrode group 31G, a second DC electrode group 32G, a distal-side potential-measuring electrode group 33G, a proximal-side potential-measuring electrode group 34G, a first lead wire group 41G, a second lead wire group 42G, a third lead wire group 43G, a fourth lead wire group 44G, a first external cord 51, a second external cord 52, a third external cord 53, a first connector 61, a second connector 62 and a third connector 63.

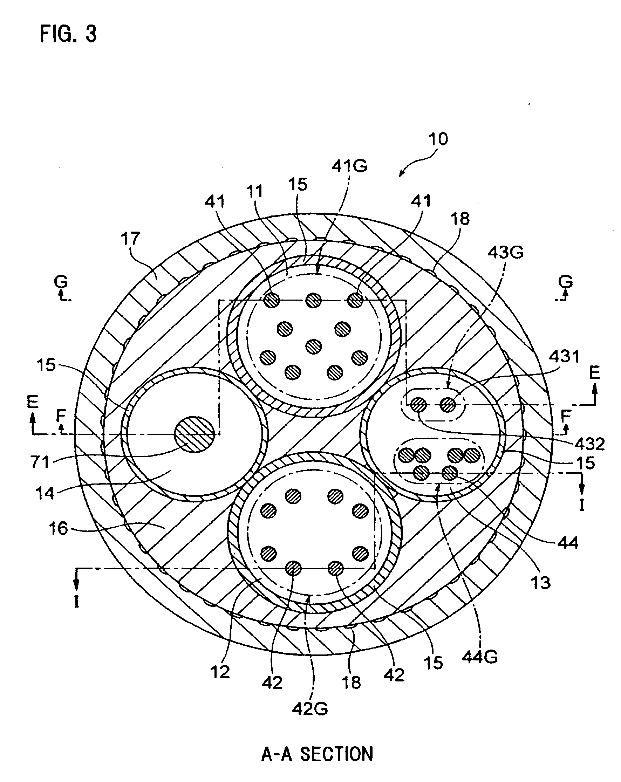

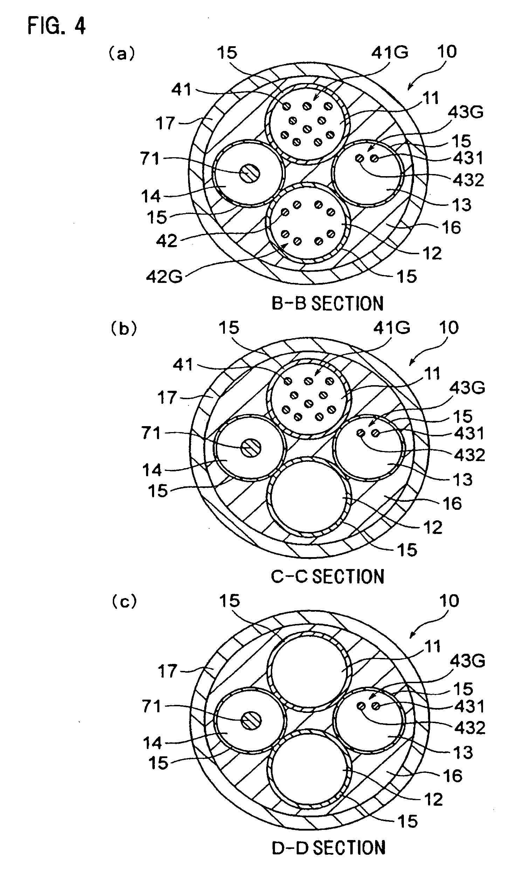

As illustrated in FIGS. 3 and 4, four lumens (a first lumen 11, a second lumen 12, a third lumen 13 and a fourth lumen 14) are formed in the multi-lumen tube 10 (an insulated tube member having a multi-lumen structure) making up the intracardiac defibrillation catheter 100 according to this embodiment.

In FIGS. 3 and 4, reference numerals 15, 16 and 17 designate a fluororesin layer partitioning into the lumens, an inner (core) part composed of a nylon elastomer having a low hardness and an outer (shell) part composed of a nylon elastomer having a high hardness, respectively. In FIG. 3, reference numeral 18 designates a stainless steel wire forming a braid.

The fluororesin layer 15 partitioning into the lumens is formed of a material having high insulating property, for example, a perfluoroalkyl vinyl ether copolymer (PFA) or polytetrafluoroethylene (PTFE).

The nylon elastomer forming the outer part 17 of the multi-lumen tube 10, having different hardnesses in an axial direction, is used, whereby the multi-lumen tube 10 is formed in such a manner that the hardness becomes higher stepwise toward the proximal side from the distal side.

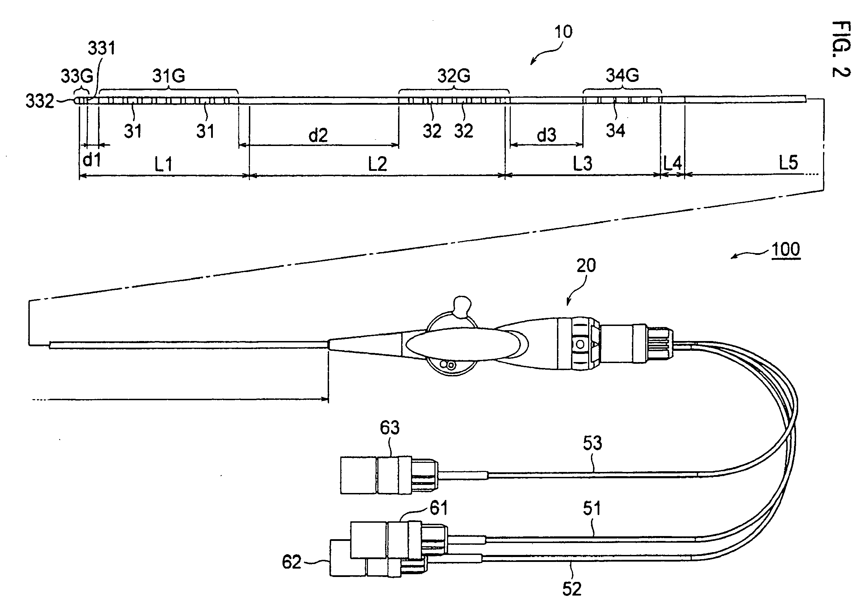

As a preferable example, the hardness (hardness as measured by a D-type hardness meter) in a region indicated by L1 (length: 65 mm) in FIG. 2 is 40, the hardness in a region indicated by L2 (length: 110 mm) is 55, the hardness in a region indicated by L3 (length: 60 mm) is 63, the hardness in a region indicated by L4 (length: 10 mm) is 68, and the hardness in a region indicated by L5 (length: 500 mm) is 72.

The braid formed by the stainless steel wire 18 is formed in only the region indicated by L5 in FIG. 2 and is provided between the inner part 16 and the outer part 17 as illustrated in FIG. 3.

The outer diameter of the multi-lumen tube 10 is, for example, 1.2 to 3.3 mm.

No particular limitation is imposed on a method for producing the multi-lumen tube 10.

The handle 20 making up the intracardiac defibrillation catheter 100 according to this embodiment is equipped with a handle body 21, a lug 22, a connector part 23 and a strain relief 24.

The lug 22 is rotationally operated, whereby a distal end portion of the multi-lumen tube 10 can be deflected (oscillated).

The first DC electrode group 31G, the second DC electrode group 32G, the distal-side potential-measuring electrode group 33G and the proximal-side potential-measuring electrode group 34G are installed on an outer periphery (a distal region where no braid is formed in the interior thereof) of the multi-lumen tube 10. Here, "the electrode group" means an assembly of a plurality of electrodes that are installed at narrow intervals (for example, 5 mm or less), and form the same pole (have the same polarity) or have the same object.

The first DC electrode group is composed of a plurality of electrodes forming the same pole (minus pole or plus pole) and installed at narrow intervals in the distal region of the multi-lumen tube. Here, the number of the electrodes making up the first DC electrode group is, for example, 8 to 12, preferably 8 to 10 though it varies according to the width of individual electrodes and arrangement interval.

In this embodiment, the first DC electrode group 31G is made up of 10 ring-like electrodes 31 installed in the distal region of the multi-lumen tube 10.

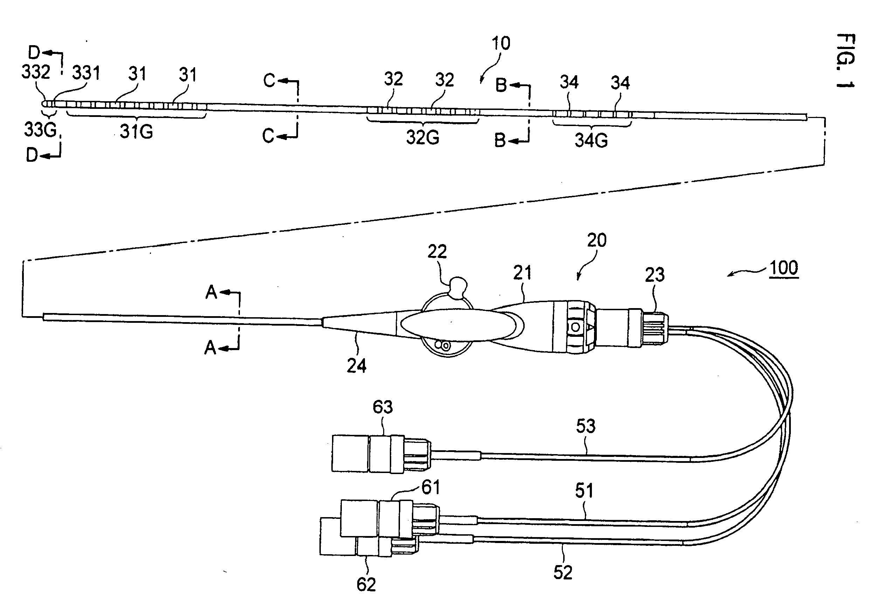

The electrodes 31 making up the first DC electrode group 31G are connected to terminals having the same pole in a direct current power unit through lead wires (lead wires 41 making up the first lead wire group 41G) and the connector (first connector 61 illustrated in FIG. 1).

Here, the width (length in an axial direction) of the electrode 31 is preferably 3 to 5 mm, and is 4 mmas a preferable example.

If the width of the electrode 31 is too narrow, the quantity of heat generated upon application of voltage becomes excessive, and so there is a possibility that a surrounding tissue may be damaged. If the width of the electrode 31 is too wide on the other hand, the flexibility or softness of a portion of the multi-lumen tube 10, in which the first DC electrode group 31G is provided, may be impaired in some cases.

An installation interval (clearance distance between adjoining electrodes) between the electrodes 31 is preferably 1 to 3 mm, and is 2 mm as a preferable example.

The first DC electrode group 31G is located in, for example, a coronary vein upon use (upon arrangement into a cardiac cavity) of the intracardiac defibrillation catheter 100.

The second DC electrode group is composed of a plurality of electrodes forming a pole (plus pole or minus pole) opposite to the first DC electrode group and installed at narrow intervals on the multi-lumen tube towards proximal direction from the installation position of the first DC electrode group. Here, the number of the electrodes making up the second DC electrode group is, for example, 6 to 10, preferably 8 to 10 though it varies according to the width of individual electrodes and arrangement interval.

In this embodiment, the second DC electrode group 32G is made up of 8 ring-like electrodes 32 installed on the multi-lumen tube 10 towards proximal direction from the installation position of the first DC electrode group 31G.

The electrodes 32 making up the second DC electrode group 32G are connected to terminals (terminals having a pole opposite to those, to which the first DC electrode group 31G is connected) having the same pole in the direct current power unit through lead wires (lead wires 42 making up the second lead wire group 42G) and the connector (second connector 62 illustrated in FIG. 1).

Voltages different in polarity from each other are thereby respectively applied to the first DC electrode group 31G (electrodes 31) and the second DC electrode group 32G (electrodes 32), and so the first DC electrode group 31G and the second DC electrode group 32G become electrode groups different in polarity from each other (when the polarity of one electrode group is minus, the polarity of the other electrode group becomes plus).

Here, the width (length in an axial direction) of the electrode 32 is preferably 3 to 5 mm, and is 4 mm as a preferable example.

If the width of the electrode 32 is too narrow, the quantity of heat generated upon application of voltage becomes excessive, and so there is a possibility that a surrounding tissue may be damaged. If the width of the electrode 32 is too wide on the other hand, the flexibility or softness of a portion of the multi-lumen tube 10, in which the second DC electrode group 32G is provided, may be impaired in some cases.

An installation interval (clearance distance between adjoining electrodes) between the electrodes 32 is preferably 1 to 3 mm, and is 2 mm as a preferable example.

The second DC electrode group 32G is located in, for example, a right atrium upon use (upon arrangement into a cardiac cavity) of the intracardiac defibrillation catheter 100.

In this embodiment, the distal-side potential-measuring electrode group 33G is made up of a ring-like electrode 331 installed on the multi-lumen tube 10 towards distal direction from the installation position of the first DC electrode group 31G, and a distal-end tip electrode 332.

The electrodes 331 and 332 making up the distal-side potential-measuring electrode group 33G are connected to an electrocardiograph through lead wires (lead wire 431 and lead wire 432 making up the third lead wire group 43G) and the connector (third connector 63 illustrated in FIG. 1). The electrode 331 is thereby clearly distinguished from the electrodes 31 connected to the direct current power unit.

Here, the width (length in an axial direction) of the electrode 331 is preferably 0.5 to 2.0 mm, and is 1.2 mm as a preferable example. The width of the electrode 332 is preferably 1.0 to 4.0 mm, and is 2 mm as a preferable example.

If the widths of the electrodes 331 and 332 are too wide, the measurement accuracy of a cardiac potential is lowered, and it is difficult to ascertain a site where an abnormal potential has been generated.

An installation interval (clearance distance) between the electrodes 331 and 332 is preferably 1.0 to 2.5 mm, and is 2 mm as a preferable example.

In this embodiment, the proximal-side potential-measuring electrode group 34G is made up of 6 ring-like electrodes 34 installed on the multi-lumen tube 10 towards proximal direction from the installation position of the second DC electrode group 32G.

The electrodes 34 making up the proximal-side potential-measuring electrode group 34G are connected to the electrocardiograph through lead wires (lead wires 44 making up the fourth lead wire group 44G) and the connector (third connector 63 illustrated in FIG. 1).

Here, the width (length in an axial direction) of the electrode 34 is preferably 0.5 to 2.0 mm, and is 1.2 mm as a preferable example.

If the width of the electrode 34 is too wide, the measurement accuracy of a cardiac potential is lowered, and it is dif f icult to ascertain a site where an abnormal potential has been generated.

An installation interval (clearance distance between adjoining electrodes) between the electrodes 34 is preferably 1. 0 to 10.0 mm, and is 5 mm as a preferable example.

The proximal-side potential- measuring electrode group 34G is located in, for example, a superior vena cava where an abnormal potential tend to generate upon use (upon arrangement into a cardiac cavity) of the intracardiac defibrillation catheter 100.

A clearance distance d1 between the distal-side potential- measuring electrode group 33G (electrode 331) and the first DC electrode group 31G (distal-side electrode 31) is preferably 0.5 to 20 mm, and is 5 mm as a preferable example.

A clearance distance d2 between the first DC electrode group 31G (proximal-side electrode 31) and the second DC electrode group 32G (distal-side electrode 32) is preferably 40 to 100 mm, and is 66 mm as a preferable example.

A clearance distance d3 between the second DC electrode group 32G (proximal-side electrode 32) and the proximal-side potential-measuring electrode group 34G (distal-side electrode 34) is preferably 5 to 50 mm, and is 30 mm as a preferable example.

The electrodes 31, 32, 331, 332 and 34 making up the first DC electrode group 31G, the second DC electrode group 32G, the distal-side potential-measuring electrode group 33G and the proximal-side potential-measuring electrode group 34G are preferably formed of platinum or a platinum-based alloy for the purpose of making radiopacity good.

The first lead wire group 41G illustrated in FIGS. 3 and 4 is an assembly of 10 lead wires 41 respectively connected to the 10 electrodes (31) making up the first DC electrode group (31G).

Each of the 10 electrodes 31 making up the first DC electrode group 31G can be electrically connected to the direct current power unit through the first lead wire group 41G (lead wire 41).

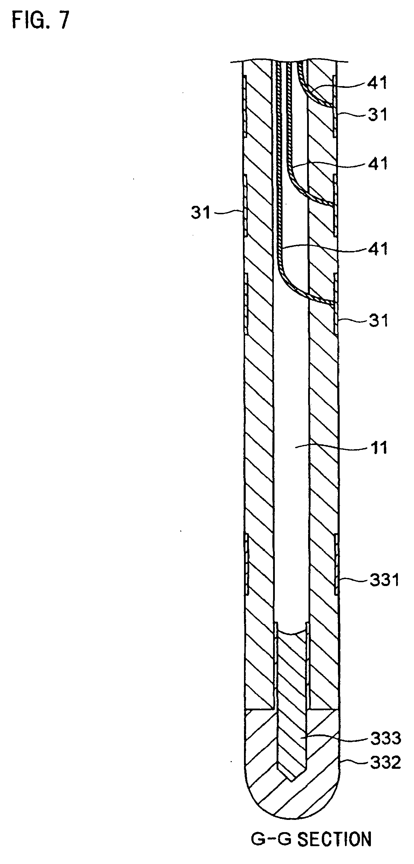

As illustrated in FIG. 7, the electrodes 31 (3 electrodes among the 10 electrodes are illustrated in FIG. 7) making up the first DC electrode group 31G are respectively connected to the separate lead wires 41. Each of the lead wires 41 is welded at its distal end portion to an inner peripheral surface of the electrode 31 and enters the first lumen 11 from a side hole (not illustrated) formed in a tube wall of the multi-lumen tube 10. The 10 lead wires 41 entered into the first lumen 11 extend into the first lumen 11 as the first lead wire group 41G.

The second lead wire group 42G illustrated in FIGS. 3 and 4 is an assembly of 8 lead wires 42 respectively connected to the 8 electrodes (32) making up the second DC electrode group (32G).

Each of the 8 electrodes 32 making up the second DC electrode group 32G can be electrically connected to the direct current power unit through the second lead wire group 42G (lead wire 42).

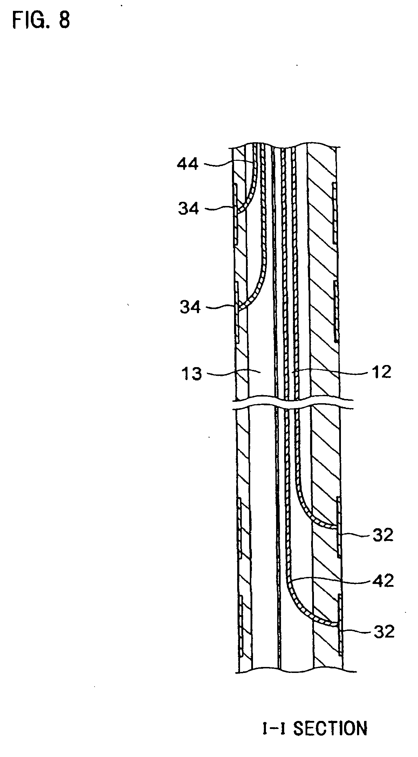

As illustrated in FIG. 8, the electrodes 32 (2 electrodes among the 8 electrodes are illustrated in FIG. 8) making up the second DC electrode group 32G are respectively connected to the separate lead wires 42. Each of the lead wires 42 is welded at its distal end portion to an inner peripheral surface of the electrode 32 and enters the second lumen 12 (lumen different from the first lumen 11 to which the first lead wire group 41G extends) from a side hole (not illustrated) formed in the tube wall of the multi-lumen tube 10. The 8 lead wires 42 entered into the second lumen 12 extend into the second lumen 12 as the second lead wire group 42G.

The first lead wire group 41G extends into the first lumen 11, and the second lead wire group 42G extends into the second lumen 12 as described above, whereby both lead wire groups are completely insulated and isolated from each other within the multi-lumen tube 10. Thus, short circuit between the first lead wire group 41G (first DC electrode group 31G) and the second lead wire group 42G (second DC electrode group 32G) can be surely prevented when the voltage necessary for the defibrillation is applied.

The third lead wire group 43G illustrated in FIGS. 3 and 4 is an assembly of the lead wire 431 connected to the ring-like electrode (331) and the lead wire 432 connected to the distal-end tip electrode (332), which make up the distal-side potential-measuring electrode group (33G).

The electrodes 331 and 332 making up the distal-side potential-measuring electrode group 33G can be respectively connected to the electrocardiograph through the third lead wire group 43G (lead wires 431 and 432).

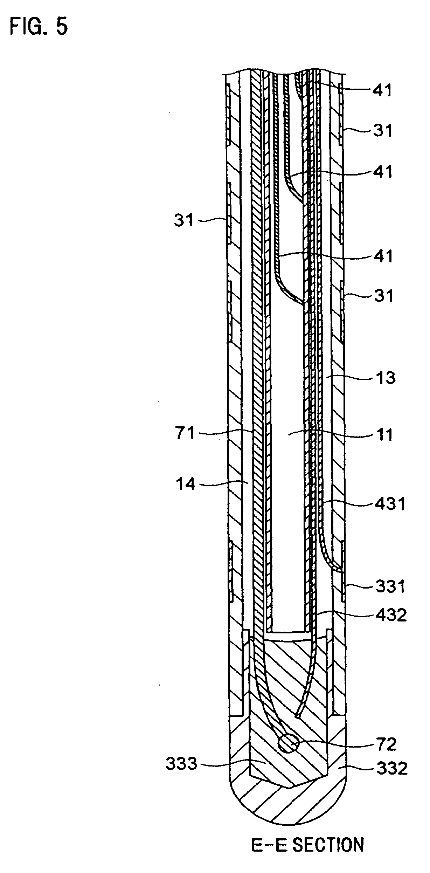

As illustrated in FIG. 5, the lead wire 431 is welded at its distal end portion to an inner peripheral surface of the electrode 331 and enters the third lumen 13 (lumen different from the first lumen 11 to which the first lead wire group 41G extends and the second lumen 12 to which the second lead wire group 42G extends) from a side hole (not illustrated) formed in the tube wall of the multi-lumen tube 10. The lead wire 432 is bonded at its distal end to the distal-end tip electrode 332 with solder 333 and enters the third lumen 13. The lead wires 431 and 432 entered into the third lumen 13 extend into the third lumen 13 as the third lead wire group 43G.

The third lead wire group 43G extending into the third lumen 13 as described above is completely insulated and isolated from both first lead wire group 41G and second lead wire group 42G. Thus, short circuit between the third lead wire group 43G (distal-side potential-measuring electrode group 33G) and the first lead wire group 41G (first DC electrode group 31G) or the second lead wire group 42G (second DC electrode group 32G) can be surely prevented when the voltage necessary for the defibrillation is applied.

The fourth lead wire group 44G illustrated in FIG. 3 is an assembly of 6 lead wires 44 respectively connected to the electrodes (34) making up the proximal-side potential-measuring electrode group (34G).

Each of the electrodes 34 making up the proximal-side potential-measuring electrode group 34G can be connected to the electrocardiograph through the fourth lead wire group 44G (lead wire 44).

As illustrated in FIG. 8, the electrodes 34 (2 electrodes among the 6 electrodes are illustrated in FIG. 8) making up the proximal-side potential-measuring electrode group 34G are respectively connected to the separate lead wires 44. Each of the lead wires 44 is welded at its distal end portion to an inner peripheral surface of the electrode 34 and enters the third lumen 13 from a side hole (not illustrated) formed in the tube wall of the multi-lumen tube 10. The 6 lead wires 44 entered into the third lumen 13 extend into the third lumen 13 as the fourth lead wire group 44G.

The fourth lead wire group 44G extending into the third lumen 13 as described above is completely insulated and isolated from both first lead wire group 41G and second lead wire group 42G. Thus, short circuit between the fourth lead wire group 44G (proximal-side potential-measuring electrode group 34G) and the first lead wire group 41G (first DC electrode group 31G) or the second lead wire group 42G (second DC electrode group 32G) can be surely prevented when the voltage necessary for the defibrillation is applied.

The lead wires 41, the lead wires 42, the lead wire 431, the lead wire 432 and the lead wires 44 are each composed of a resin-coated wire obtained by coating an outer periphery of a metal conductor with a resin such as polyimide. The coating thickness of the resin is controlled to about 5 to 10 µm.

In FIGS. 3 and 4, reference numeral 71 designates a pull wire.

The pull wire 71 extends into the fourth lumen 14 and located eccentrically from a central axis of the multi-lumen tube 10.

As illustrated in FIG. 5, a distal end portion of the pull wire 71 is fixed to the distal-end tip electrode 332 with solder 333. A large-diameter portion (retaining portion) 72 for fall stopping is formed at the tip of the pull wire 71. The distal-end tip electrode 332 can be thereby firmly bonded to the pull wire 71 to surely prevent the distal-end tip electrode 332 from falling.

On the other hand, a proximal end portion of the pull wire 71 is connected to the lug 22 of the handle 20, and the pull wire 71 is pulled by operating the lug 22, whereby a distal end portion of the multi-lumen tube 10 is deflected.

The pull wire 71 is formed by a stainless steel or Ni-Ti-based super-elastic alloy. However, the pull wire may not necessarily formed by a metal. The pull wire 71 may be formed by, for example, a nonconductive wire having high strength.

Incidentally, the mechanism for deflecting the distal end portion of the multi-lumen tube is not limited to this, and it may be constructed by providing, for example, a plate spring.

Only the pull wire 71 extends into the fourth lumen 14 of the multi-lumen tube 10, and no lead wire (group) extends. The lead wires can thereby be prevented from being damaged (for example, abraded) by the pull wire 71 moving in the axial direction upon the distal end deflection operation of the multi-lumen tube 10.

In the intracardiac defibrillation catheter 100 according to this embodiment, the first lead wire group 41G, the second lead wire group 42G, and the third lead wire group 43G and the fourth lead wire group G are insulated and isolated from one another even in the interior of the handle 20.

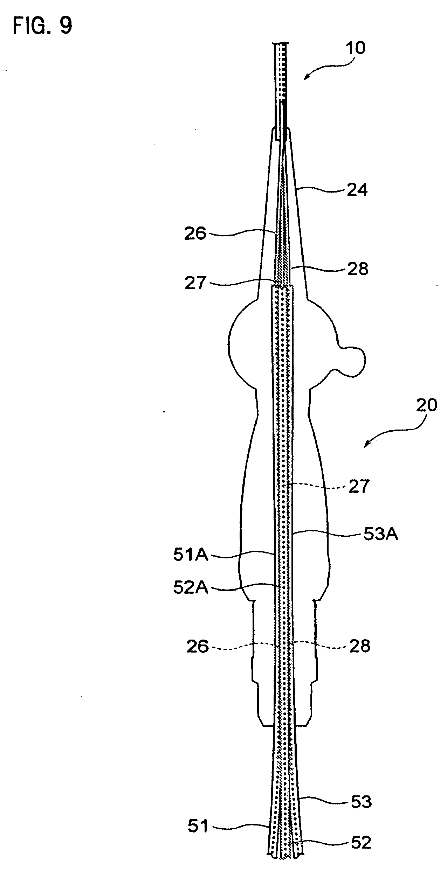

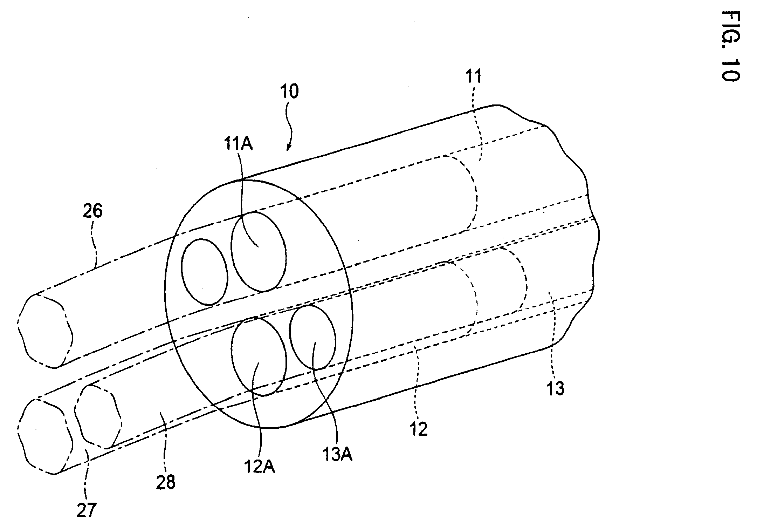

FIG. 9 is an explanatory view typically illustrating the interior of the handle 20 making up the intracardiac defibrillation catheter 100, and FIG. 10 is an explanatory view illustrating, on an enlarged scale, a proximal end portion of the multi-lumen tube 10 connected to the handle 20. Incidentally, the lead wire groups and the pull wire are omitted from illustration in FIGS. 9 and 10.

As illustrated in FIG. 9, the proximal end portion of the multi-lumen tube 10 is inserted into a distal end opening of the handle 20 (strain relief 24), whereby the multi-lumen tube 10 is connected to the handle 20.

In the interior of the handle 20, 3 insulated tubes (first insulated tube 26, second insulated tube 27 and third insulated tube 28), into which the respective lead wire groups are inserted, extend.

As illustrated in FIG. 10, a distal end portion (portion about 10 mm long from the tip) of the first insulated tube 26 is inserted into the first lumen 11 from an opening 11A, whereby the first insulated tube 26 is connected to the first lumen 11 into which the first lead wire group extends.

The first insulated tube 26 connected to the first lumen 11 passes through an inner hole of a first protecting tube 51A extending in the interior of the handle 20 and extends to a connector (not illustrated, the first connector 61 illustrated in FIG. 1) located outside the handle 20 to form a passage through which the proximal end portion of the first lead wire group is guided to the connector.

The first lead wire group extended out from the multi-lumen tube 10 thereby extends into the first insulated tube 26 and is connected to the connector (first connector 61).

Incidentally, the first protecting tube 51A, into which the first insulated tube 26 is inserted, extends from the handle 20 to the outside to form an external cord (first external cord 51 illustrated in FIG. 1).

As illustrated in FIG. 10, a distal end portion (portion about 10 mm long from the tip) of the second insulated tube 27 is inserted into the second lumen 12 from an opening 12A, whereby the second insulated tube 27 is connected to the second lumen 12 into which the second lead wire group extends.

The second insulated tube 27 connected to the second lumen 12 passes through an inner hole of a second protecting tube 52A extending in the interior of the handle 20 and extends to a connector (not illustrated, the second connector 62 illustrated in FIG. 1) located outside the handle 20 to form a passage through which the proximal end portion of the second lead wire group is guided to the connector.

The second lead wire group extended out from the multi-lumen tube 10 thereby extends into the second insulated tube 27 and is connected to the connector (second connector 62).

Incidentally, the second protecting tube 52A, into which the second insulated tube 27 is inserted, extends from the handle 20 to the outside to form an external cord (second external cord 52 illustrated in FIG. 1).

As illustrated in FIG. 10, a distal end portion (portion about 10 mm long from the tip) of the third insulated tube 28 is inserted into the third lumen 13 from an opening 13A, whereby the third insulated tube 28 is connected to the third lumen 13 into which the third lead wire group and the fourth lead wire group extend.

The third insulated tube 28 connected to the third lumen 13 passes through an inner hole of a third protecting tube 53A extending in the interior of the handle 20 and extends to a connector (not illustrated, the third connector 63 illustrated in FIG. 1) located outside the handle 20 to form a passage through which the proximal end portions of the third lead wire group and fourth lead wire group are guided to the connector.

The third lead wire group and fourth lead wire group extended out from the multi-lumen tube 10 thereby extend into the third insulated tube 28 and is connected to the connector (third connector 63).

Incidentally, the third protecting tube 53A, into which the third insulated tube 28 is inserted, extends out of the handle 20 to form an external cord (third external cord 53 illustrated in FIG. 1).

As examples of materials forming the insulated tubes (first insulated tube 26, second insulated tube 27 and third insulated tube 28), may be mentioned polyimide resins, polyamide resins and polyamide-imide resins. The wall thickness of each insulated tube is preferably 20 to 40 µm, and is 30 µm as a preferable example.

As examples of materials forming the protecting tubes (first protecting tube 51A, second protecting tube 52A and third protecting tube 53A), into which the insulated tubes are respectively inserted, may be mentioned nylon-based elastomers such as "Pebax" (product of ARKEMA CO.).

According to the intracardiac defibrillation catheter 100 of this embodiment having such a structure as described above, the first lead wire group 41G extends into the first insulated tube 26, the second lead wire group 42G extends into the second insulated tube 27, and the third lead wire group 43G and the fourth lead wire group 44G extend into the third insulated tube 28, whereby the first lead wire group 41G, the second lead wire group 42G, and the third lead wire group 43G and the fourth lead wire group 44G can be completely insulated and isolated from one another even in the interior of the handle 20. As a result, short circuit (in particular, short circuit between lead wire groups extended out in the vicinity of the openings of the lumens) between the first lead wire group 41G, the second lead wire group 42G and the third lead wire group 43G or the fourth lead wire group 44G in the interior of the handle 20 can be surely prevented when the voltage necessary for the defibrillation is applied.

In addition, the insulated tubes (first insulated tube 26, second insulated tube 27 and third insulated tube 28) are respectively protected by the protecting tubes (first protecting tube 51A, second protecting tube 52A and third protecting tube 53A), whereby the insulated tubes can be prevented from being damaged by, for example, contact or abrasion with a member of the lug 22 upon a distal end deflection operation of the multi-lumen tube 10.

In the intracardiac defibrillation catheter 100 according to this embodiment, each of the 10 electrodes 31 making up the first DC electrode group 31G is connected to a terminal of one pole in the direct current power unit through each of the 10 lead wires 41 making up the first lead wire group 41G and the first connector 61.

On the other hand, each of the 8 electrodes 32 making up the second DC electrode group 32G is connected to a terminal of the other pole in the direct current power unit through each of the 8 lead wires 42 making up the second lead wire group 42G and the second connector 62.

Further, the 2 electrodes (electrode 331 and electrode 332) making up the distal-side potential-measuring electrode group 33G are connected to the electrocardiograph through the 2 lead wires (lead wire 431 and lead wire 432) making up the third lead wire group 43G and the third connector 63.

Furthermore, the 6 electrodes 34 making up the proximal-side potential-measuring electrode group 34G are connected to the electrocardiograph through the 6 lead wires 44 making up the fourth lead wire group 44G and the third connector 63.

The intracardiac defibrillation catheter 100 according to this embodiment is a catheter used for applying a direct current voltage between the first DC electrode group 31G and the second DC electrode group 32G, thereby directly applying electric energy to a heart that has suffered fibrillation to conduct a defibrillation treatment, and is different from the conventionally known electrode catheter used in diagnosis of arrhythmia (measurement of cardiac potential) or a cauterization treatment in use and function.

The intracardiac defibrillation catheter 100 according to this embodiment is suitably used upon cardiac catheterization during which atrial fibrillation is liable to occur. Particularly preferably, the intracardiac defibrillation catheter 100 is inserted into a cardiac cavity of a patient in advance to conduct the cardiac catheterization.

The intracardiac defibrillation catheter 100 is inserted into a cardiac cavity in such a manner that the first DC electrode group 31G is located in a coronary vein, and the second DC electrode group 32G is located in a right atrium, thereby creating a state that the heart is held between the first DC electrode group 31G and the second DC electrode group 32G.

An electrocardiogram measured by the distal-side potential-measuring electrode group 33G or the proximal-side potential-measuring electrode group 34G during cardiac catheterization is monitored to stop the cardiac catheterization when atrial fibrillation has occurred so as to conduct a defibrillation treatment by the intracardiac defibrillation catheter 100. Specifically, a direct current voltage is applied between the first DC electrode group 31G and the second DC electrode group 32G through the first lead wire group 41G and the second lead wire group 42G to directly give electric energy to the heart that has suffered the fibrillation.

Here, the electric energy supplied to the heart by the intracardiac defibrillation catheter 100 is preferably 10 to 30 J.

If the electric energy is too small, it is impossible to sufficiently conduct the defibrillation treatment. If the electric energy is excessive on the other hand, tissues about the first DC electrode group 31G and the second DC electrode group 32G may possibly be damaged.

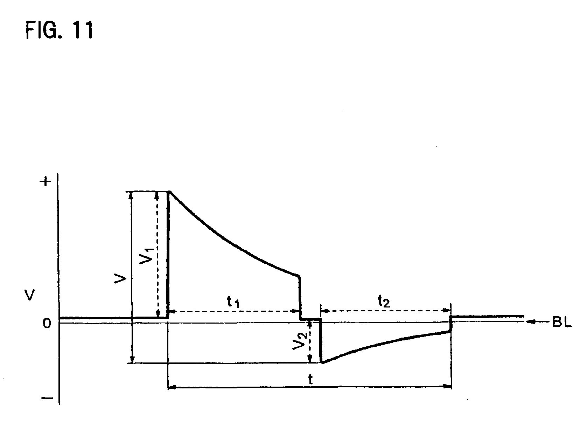

FIG. 11 is a potential waveform diagram measured when predetermined electric energy (for example, setting output = 10 J) has been applied by the intracardiac defibrillation catheter 100 according to this embodiment. In this diagram, an axis of abscissa and an axis of ordinate indicate a time and a potential, respectively.

First, a direct current voltage is applied between the first DC electrode group 31G and the second DC electrode group 32G in such a manner that the first DC electrode group 31G becomes a minus pole and the second DC electrode group 32G becomes a plus pole, whereby electric energy is supplied to build up a measuring potential (V₁ is a peak voltage at this time). After a fixed time (t₁) has elapsed, a direct current voltage that the poles have been reversed in such a manner that the first DC electrode group 31G becomes a plus pole and the second DC electrode group 32G becomes a minus pole is applied between both electrode groups, whereby electric energy is supplied to build up a measuring potential (V₂ is a peak voltage at this time).

Here, the time (t₁) is, for example, 1.5 to 10.0 seconds, and the peak voltage (V₁) measured is, for example, 300 to 500 V.

In the intracardiac defibrillation catheter 100 according to this embodiment, high electric energy is supplied (high voltage is applied) though it is low compared with AED, so that it is necessary to surely prevent short circuit, which has not become a problem in the conventional electrode catheter, to ensure safety.

Thus, in the intracardiac defibrillation catheter 100, the first lead wire group 41G connected to the first DC electrode group 31G is caused to extend into the first lumen 11 formed in the multi-lumen tube 10 and into the first insulated tube 26 in the interior of the handle 20 to be connected to the first connector 61, the second lead wire group 42G connected to the second DC electrode group 32G is caused to extend into the second lumen 12 formed in the multi-lumen tube 10 and into the second insulated tube 27 in the interior of the handle 20 to be connected to the second connector 62, and the third lead wire group 43G connected to the distal-side potential-measuring electrode group 33G and the fourth lead wire group 44G connected to the proximal-side potential-measuring electrode group 34G are respectively caused to extend into the third lumen 13 formed in the multi-lumen tube 10 and into the third insulated tube 28 in the interior of the handle 20 to be connected to the third connector 63.

The first lead wire group 41G, the second lead wire group 42G, and the third lead wire group 43G and the fourth lead wire group 44G can thereby be completely insulated and isolated from one another in the interior of the multi-lumen tube 10 and the interior of the handle 20.

Accordingly, short circuit among the first lead wire group 41G (first DC electrode group 31G), the second lead wire group 42G (second DC electrode group 32G) and the third lead wire group 43G or the fourth lead wire group 44G (distal-side potential-measuring electrode group 33G or proximal-side potential-measuring electrode group 34G) can be surely prevented when the voltage necessary for the defibrillation is applied.

One embodiment of the present invention has been described above. However, the intracardiac defibrillation catheter according to the present invention is not limited thereto, and various modifications may be made.

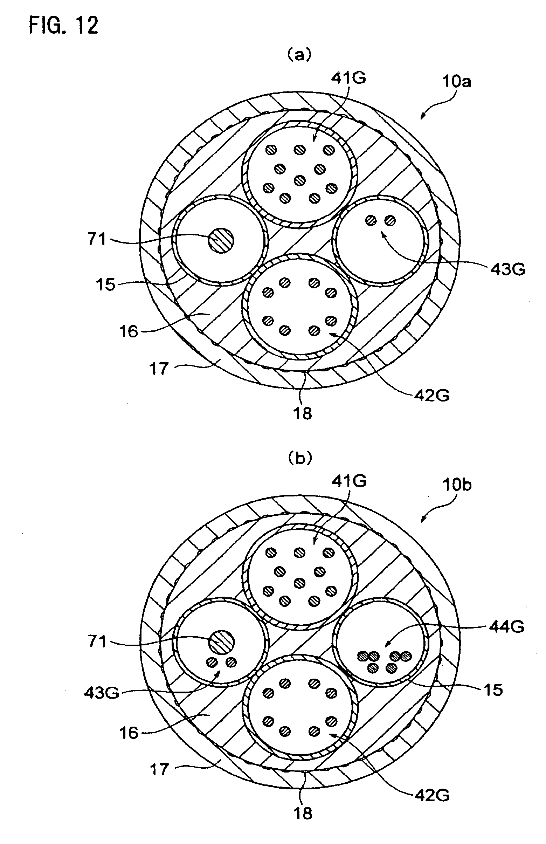

For example, only the distal-side potential-measuring electrode group 33G may be provided as a potential-measuring electrode group, and only the third lead wire group 43G may extend into the third lumen 13 as a lead wire group for potential measurement as illustrated in FIG. 12(a).

Alternatively, the third lead wire group 4 3G may extend into the fourth lumen 14 together with the pull wire 71, and the fourth lead wire group 44G may extend into the third lumen 13 as illustrated in FIG. 12(b).

Further, only the proximal-side potential-measuring electrode group may be provided as a potential-measuring electrode group, and only the fourth lead wire group may extend into the third lumen 13 as a lead wire group for potential measurement.

### DESCRIPTION OF CHARACTERS

- 100: Intracardiac defibrillation catheter
- 10: Multi-lumen tube
- 11: First lumen
- 12: Second lumen
- 13: Third lumen
- 14: Fourth lumen
- 15: Fluororesin layer
- 16: Inner (core) part
- 17: Outer (shell) part
- 18: Stainless steel wire
- 20: Handle
- 21: Handle body
- 22: Lug
- 23: Connector part
- 24: Strain relief
- 26: First insulated tube
- 27: Second insulated tube
- 28: Third insulated tube
- 31G: First DC electrode group
- 31: Ring-like electrodes
- 32G: Second DC electrode group
- 32: Ring-like electrodes
- 33G: Distal-side potential-measuring electrode group
- 331: Ring-like electrode
- 332: Distal end tip electrode
- 333: Solder
- 34G: Proximal-side potential-measuring electrode group
- 34: Ring-like electrodes
- 41G: First lead wire group
- 41: Lead wires
- 42G: Second lead wire group
- 42: Lead wires
- 43G: Third lead wire group
- 431: Lead wire
- 432: Lead wire
- 44G: Fourth lead wire group
- 44: Lead wires
- 51: First external cord
- 52: Second external cord
- 53: Third external cord
- 51A: First protecting tube
- 52A: Second protecting tube
- 53A: Third protecting tube
- 61: First connector
- 62: Second connector
- 63: Third connector
- 71: Pull wire
- 72: Large-diameter portion for fall stopping

## Claims

1. An intracardiac defibrillation catheter (100) inserted into a cardiac cavity to conduct defibrillation, the catheter (100) comprising
an insulated tube member (10) having a multi-lumen structure,
a handle (20) connected to a proximal end of the tube member (10),
a first defibrillation electrode group (31G) composed of a plurality of ring-like defibrillation electrodes (31) and installed in a distal region of the tube member (10) and adapted for connection to a direct current unit,
a second defibrillation electrode group (32G) composed of a plurality of ring-like defibrillation electrodes (32) and installed on the tube member (10) towards proximal direction from the first defibrillation electrode group (31 G) and adapted for connection to a direct current unit,
a first lead wire group (41G) composed of lead wires (41) connected to the respective defibrillation electrodes (31) making up the first defibrillation electrode group (31G), and a second lead wire group (42G) composed of lead wires (42) connected to the respective defibrillation electrodes (32) making up the second defibrillation electrode group (32G), wherein
the first lead wire group (41G) and the second lead wire group (42G) respectively extend into different lumens (11, 12) of the tube member (10), and
voltages different in polarity from each other are respectively applied to the first defibrillation electrode group (31 G) and the second defibrillation electrode group (32G) when defibrillation is conducted,
the intracardiac defibrillation catheter (100) being **characterized by**
a distal-side potential-measuring electrode group (33G) composed of a plurality of ring-like electrodes (331, 332) and installed on the tube member (10) towards distal direction from the first electrode group (31G) and adapted for connection to an electrocardiograph,
a proximal-side potential-measuring electrode group (34G) composed of a plurality of ring-like electrodes (34) and installed on the tube member (10) towards proximal direction from the second electrode group (32G) and adapted for connection to an electrocardiograph,
a third lead wire group (43G) composed of lead wires (43) connected to the respective electrodes (331, 332) making up the distal-side potential-measuring electrode group (33G), and
a fourth lead wire group (44G) composed of lead wires (44) connected to the respective electrodes (34) making up the proximal-side potential-measuring electrode group (34G),
wherein
the third lead wire group (43G) and the fourth lead wire group (44G) extend into any other lumen (13) of the tube member (10) than both lumens (11, 12) into which the first lead wire group (41 G) and the second lead wire group (42G) extend.

2. The intracardiac defibrillation catheter (100) according to claim 1, wherein
4 lumens (11, 12, 13, 14) are formed in the tube member (10),
the first lead wire group (41G) extends into a first lumen (11),
the second lead wire group (42G) extends into a second lumen (12),
the third lead wire group (43G) and fourth lead wire group (44G) extend into a third lumen (13), and
a pull wire (71) for distal end deflection operation extends into a fourth lumen (14).

3. The intracardiac defibrillation catheter (100) according to any one of claims 1 or 2, wherein the first lead wire group (41G) and the second lead wire group (42G) extend into inner holes of insulated tubes (26, 27, 28) different from each other in the interior of the handle (20).

4. The intracardiac defibrillation catheter (100) according to claim 2, wherein in the interior of the handle (20),
the first lead wire group (41G) extends into a first insulated tube (26) connected to the first lumen (11),
the second lead wire group (42G) extends into a second insulated tube (27) connected to the second lumen (12), and
the third lead wire group (43G) and the fourth lead wire group (44G) extend into a third insulated tube (28) connected to the third lumen (13).

5. The intracardiac defibrillation catheter (100) according to claim 1, wherein the defibrillation electrodes (31, 32) of at least one of the first and the second defibrillation electrode groups (31G, 32G) have a width of 3 to 5 mm.

6. The intracardiac defibrillation catheter (100) according to one of the preceding claims, wherein the electrodes of at least one of the first electrode group (31G), the second electrode group (32G), the distal-side potential-measuring electrode group (33G) and the proximal-side potential-measuring electrode group (34G) are installed at intervals of 5 mm or less.

7. The intracardiac defibrillation catheter (100) according to claim 1, wherein the first defibrillation electrode group (31G) comprises 8 to 12 electrodes (31).

8. The intracardiac defibrillation catheter (100) according to claim 1, wherein the second defibrillation electrode group (32G) comprises 6 to 10 electrodes (32).

## Patentansprüche

1. Defibrillations-Herzkatheter (100), der zur Ausführung einer Defibrillation in eine Herzkavität eingeführt ist, wobei der Katheter (100) aufweist
ein isoliertes Rohrelement (10) mit einem Mehr-Lumen-Aufbau,
einem Griff (20), der mit einem proximal gelegenen Ende des Rohrelements (10) verbunden ist,
eine erste Defibrillations-Elektrodengruppe (31 G), die aus mehreren ringartigen Defibrillations-Elektroden (31) zusammengesetzt und in einem distal gelegenen Gebiet des Rohrelements (10) montiert und zur Verbindung mit einer Gleichstromeinheit ausgebildet ist,
eine zweite Defibrillations-Elektrodengruppe (32G), die aus mehreren ringartigen Defibrillations-Elektroden (32) zusammengesetzt und auf dem Rohrelement (10) zur proximalen Richtung weisend ausgehend von der ersten Defibrillations-Elektrodengruppe (31 G) montiert und zur Verbindung mit einer Gleichstromeinheit ausgebildet ist,
eine erste Anschlussleitungsgruppe (41 G), die aus Anschlussleitungen (41) zusammengesetzt ist, die mit dem jeweiligen Defibrillatorions-Elektroden (31) verbunden sind, die die erste Defibrillations-Elektrodengruppe (31 G) bilden, und
eine zweite Anschlussleitungsgruppe (42G), die aus Anschlussleitungen (42) zusammengesetzt ist, die mit den jeweiligen Defibrillations-Elektroden (32) verbunden sind, die die zweite Defibrillations-Elektrodengruppe (32G) bilden, wobei
die erste Anschlussleitungsgruppe (41 G) und die zweite Anschlussleitungsgruppe (42G) sich entsprechend in unterschiedliche Lumen (11, 12) des Rohrelements (10) erstrecken, und
Spannungen mit zueinander unterschiedlicher Polarität entsprechend an die erste Defibrillations-Elektrodengruppe (31 G) und die zweite Defibrillations-Elektrodengruppe (32G) bei Ausführung einer Defibrillations angelegt werden;
wobei der Defibrillations-Herzkatheter (100) **gekennzeichnet ist durch** eine distalseitige potenzialmessende Elektrodengruppe (33D), die aus mehreren ringartigen Elektroden (331, 332) zusammengesetzt ist und auf dem Rohrelement (10) in der distalen Richtung ausgehend von der ersten Elektrodengruppe (31 G) montiert und zur Verbindung mit einem Elektrokardiograf ausgebildet ist,
eine proximalseitige potenzialmessende Elektrodengruppe (34G), die aus mehreren ringartigen Elektroden (34) zusammengesetzt und auf dem Rohrelement (10) in proximaler Richtung ausgehend von der zweiten Elektrodengruppe (32G) montiert und zur Verbindung mit einem Elektrokariograf ausgebildet ist,
eine dritte Anschlussleitungsgruppe (43G), die aus Anschlussleitungen (43) zusammengesetzt ist, die mit den jeweiligen Elektroden (331, 332) verbunden sind, die die distalseitige potenzialmessende Elektrodengruppe (33G) bilden, und
eine vierte Anschlussleitungsgruppe (44G), die aus Anschlussleitungen (44) zusammengesetzt ist, die mit den jeweiligen Elektroden (34) verbunden sind, die die proximalseitige potenzialmessende Elektrodengruppe (34G) bilden, wobei
die dritte Anschlussleitungsgruppe (43G) und die vierte Anschlussleitungsgruppe (44G) sich in ein anderes Lumen (13) des Rohrelements (10) außer den beiden Lumen (11, 12) erstrecken, in die sich die erste Anschlussleitungsgruppe (41 G) und die zweite Anschlussleitungsgruppe (42G) erstrecken.

2. Defibrillations-Herzkatheter (100) nach Anspruch 1, wobei
4 Lumen (11, 12, 13, 14) in dem Rohrelement (10) ausgebildet sind,
die erste Anschlussleitungsgruppe (41G) sich in ein erstes Lumen (11) erstreckt,
die zweite Anschlussleitungsgruppe (42G) sich in ein zweites Lumen (12) erstreckt,
die dritte Anschlussleitungsgruppe (43G) und die vierte Anschlussleitungsgruppe (44G) sich in ein drittes Lumen (13) erstrecken, und
eine Zugleitung (71) für einen Biegevorgang am distalen Ende sich in ein viertes Lumen (14) erstreckt.

3. Defibrillations-Herzkatheter (100) nach einem der Ansprüche 1 oder 2, wobei die erste Anschlussleitungsgruppe (41G) und die zweite Anschlussleitungsgruppe (42G) sich in Innenbohrungen von zueinander unterschiedlichen isolierten Rohren (26, 27, 28) im Inneren des Griffs (20) erstrecken.

4. Defibrillations-Herzkatheter (100) nach Anspruch 2, wobei in dem Inneren des Griffs (20)
die erste Anschlussleitungsgruppe (41 G) sich in ein erstes isoliertes Rohr (26) erstreckt, das mit dem ersten Lumen (11) verbunden ist,
die zweite Anschlussleitungsgruppe (42G) sich in ein zweites isoliertes Rohr (27) erstreckt, das mit dem zweiten Lumen (12) verbunden ist, und
die dritte Anschlussleitungsgruppe (43G) und die vierte Anschlussleitungsgruppe (44G) sich in ein drittes isoliertes Rohr (28) erstrecken, das mit dem dritten Lumen (13) verbunden ist.

5. Defibrillatorion-Herzkatheter (100) nach Anspruch 1, wobei die Defibrillations-Elektroden (31, 32) der ersten und/oder der zweiten Defibrillations-Elektrodengruppe (31 G, 32G) eine Breite von 3 bis 5 mm haben.

6. Defibrillations-Herzkatheter (100) nach einem der vorhergehenden Ansprüche, wobei die Elektroden der ersten Elektrodengruppe (31 G) und/oder der zweiten Elektrodengruppe (32G) und/oder der distalseitigen potenzialmessenden Elektrodengruppe (33G) und/oder der proximalseitigen potenzialmessenden Elektrodengruppe (34G) mit Abständen von 5 mm oder weniger montiert sind.

7. Defibrillations-Herzkatheter (100) nach Anspruch 1, wobei die erste Defibrillations-Elektrodengruppe (31G) 8 bis 12 Elektroden (31) umfasst.

8. Defibrillations-Herzkatheter (100) nach Anspruch 1, wobei die zweite Defibrillations-Elektrodengruppe (32G) 6 bis 10 Elektroden (32) umfasst.

## Revendications

1. Cathéter de défribrillation intracardiaque (100) inséré dans une cavité cardiaque pour conduire la défibrillation, le cathéter (100) comprenant
un élément formant tube isolé (10) présentant une structure multi-lumière,
une poignée (20) connectée à une extrémité proximale de l'élément formant tube (10),
un premier groupe d'électrodes de défibrillation (31G) composé d'une pluralité d'électrodes de défibrillation de type anneau (31) et installé dans une région distale de l'élément formant tube (10) et adapté pour la connexion à une unité d'alimentation en courant direct,
un second groupe d'électrodes de défibrillation (32G) composé d'une pluralité d'électrodes de défibrillation de type anneau (32) et installé sur l'élément formant tube (10) vers le sens proximal depuis le premier groupe d'électrodes de défibrillation (31G) et adapté pour la connexion à une unité d'alimentation en courant direct,
un premier groupe de fils conducteurs (41G) composé de fils conducteurs (41) connectés aux électrodes respectives de défibrillation (31) constituant le premier groupe d'électrodes de défibrillation (31G), et
un second groupe de fils conducteurs (42G) composé de fils conducteurs (42) connectés aux électrodes respectives de défibrillation (32) constituant le second groupe d'électrodes de défibrillation (32G), où
le premier groupe de fils conducteurs (41G) et le second groupe de fils conducteurs (42G) s'étendent respectivement dans des lumières différentes (11, 12) de l'élément formant tube (10), et
des tensions différentes en termes de polarité l'une par rapport à l'autre sont respectivement appliquées au premier groupe d'électrodes de défibrillation (31G) et au second groupe d'électrodes de défibrillation (32G) lorsque la défibrillation est conduite,
le cathéter de défribrillation intracardiaque (100) étant **caractérisé par**
un groupe d'électrodes de mesure de potentiel côté distal (33G) composé d'une pluralité d'électrodes de type anneau (331, 332) et installé sur l'élément formant tube (10) vers le sens distal depuis le premier groupe d'électrodes (31G) et adapté pour la connexion à un électrocardiographe,
un groupe d'électrodes de mesure de potentiel côté proximal (34G) composé d'une pluralité d'électrodes de type anneau (34) et installé sur l'élément formant tube (10) vers le sens proximal depuis le second groupe d'électrodes (32G) et adapté pour la connexion à un électrocardiographe,
un troisième groupe de fils conducteurs (43G) composé de fils conducteurs (43) connectés aux électrodes respectives (331, 332) constituant le groupe d'électrodes de mesure de potentiel côté distal (33G),
et
un quatrième groupe de fils conducteurs (44G) composé de fils conducteurs (44) connectés aux électrodes respectives (34) constituant le groupe d'électrodes de mesure de potentiel côté proximal (34G), où
le troisième groupe de fils conducteurs (43G) et le quatrième groupe de fils conducteurs (44G) s'étendent dans n'importe quelle autre lumière (13) de l'élément formant tube (10) que les deux lumières (11, 12) dans lesquelles le premier groupe de fils conducteurs (41G) et le second groupe de fils conducteurs (42G) s'étendent.

2. Cathéter de défribrillation intracardiaque (100) selon la revendication 1, où
4 lumières (11, 12, 13, 14) sont formées dans l'élément formant tube (10),
le premier groupe de fils conducteurs (41G) s'étend dans une première lumière (11),
le second groupe de fils conducteurs (42G) s'étend dans une seconde lumière (12),
le troisième groupe de fils conducteurs (43G) et le quatrième groupe de fils conducteurs (44G) s'étendent dans une troisième lumière (13), et
un fil de traction (71) pour l'opération de déflexion de l'extrémité distale s'étend dans une quatrième lumière (14).

3. Cathéter de défribrillation intracardiaque (100) selon l'une quelconque des revendications 1 ou 2, où le premier groupe de fils conducteurs (41G) et le second groupe de fils conducteurs (42G) s'étendent dans des trous internes des tubes isolés (26, 27, 28) différents l'un de l'autre dans l'intérieur de la poignée (20).

4. Cathéter de défribrillation intracardiaque (100) selon la revendication 2, où dans l'intérieur de la poignée (20),
le premier groupe de fils conducteurs (41G) s'étend dans un premier tube isolé (26) connecté à la première lumière (11),
le second groupe de fils conducteurs (42G) s'étend dans un second tube isolé (27) connecté à la seconde lumière (12), et
le troisième groupe de fils conducteurs (43G) et le quatrième groupe de fils conducteurs (44G) s'étendent dans un troisième tube isolé (28) connecté à la troisième lumière (13).

5. Cathéter de défribrillation intracardiaque (100) selon la revendication 1, où les électrodes de défibrillation (31, 32) d'au moins l'un du premier et du second groupes d'électrodes de défibrillation (31G, 32G) présente une largeur de 3 à 5 mm.

6. Cathéter de défribrillation intracardiaque (100) selon l'une quelconque des revendications précédentes, où les électrodes d'au moins l'un du premier groupe d'électrodes (31G), du second groupe d'électrodes (32G), du groupe d'électrodes de mesure de potentiel côté distal (33G) et du groupe d'électrodes de mesure de potentiel côté proximal (34G) sont installées à des intervalles de 5 mm ou moins.

7. Cathéter de défribrillation intracardiaque (100) selon la revendication 1, où le premier groupe d'électrodes de défibrillation (31G) comprend de 8 à 12 électrodes (31).

8. Cathéter de défribrillation intracardiaque (100) selon la revendication 1, où le second groupe d'électrodes de défibrillation (32G) comprend de 6 à 10 électrodes (32).
